# EUROPEAN PATENT APPLICATION

(11) **EP 1 013 664 A1**
(43) Date of publication of application: **28.06.2000**
(21) Application number: 98124591.3
(22) Date of filing: 23.12.1998
(51) Int. Cl.: C07K 7/08, C07K 7/06, G01N 33/50, A61K 47/48, G01N 33/94

(54) **Antigenic determinants for drug-specific T lymphocytes from drug-allergic patients**

(71) Applicant: Max-Planck-Gesellschaft zur Förderung der Wissenschaften e.V., 80539 München (DE)
(72) Inventor: The designation of the inventor has not yet been filed
(74) Representative: Böhm, Brigitte, Dipl.-Chem. Dr.

(57) **Abstract**

Hapten-modified peptides according to the present invention contain a backbone of amino acids wherein on at least one of these amino acids a penicillin antibiotic is bound. Such hapten-modified peptides are useful as pharmaceutical compounds and/or diagnostic tools.

## Description

The present invention relates to hapten-modified peptides for use as pharmaceutical compounds and/or diagnostic tools, use of hapten-modified peptides for the diagnostic determination of a predisposition for hypersensitivity reactions, for the desensitization of patients suffering from hypersensitivity reactions against penicillins or parts or derivatives thereof, and use of penicillins as therapeutic agents for the treatment of auto immune diseases or other pathological situations where IFNγ-mediated effects are involved in the progression of the disease.

The differentiation of CD4⁺ T lymphocytes into TH1 and TH2 cells upon antigenic stimulation plays a critical role in adverse immune responses including drug-specific hypersensitivity reactions. Allergic reactions to antibiotics are frequent and diversified to represent a serious problem in practical medicine. It becomes more and more evident that human drug-specific T cells are the major players in eliciting those responses. As source of different cytokines, they are crucial either in the induction phase of IgE antibody release by producing IL4, as well as in both, the induction and the effector stages of T cell mediated reactions (Weltzien et al, Toxicology 107 (1995), 141-152; Padovan et al, Eur. J. Immunol. 26 (1996), 42-48).

Recent advances in understanding the molecular mechanisms inducing these adverse immune responses point to hapten-modified peptides as antigens (Weltzien and Padovan, J. Invest. Dermatol. 110 (1998), 203-206; Padovan et al, Eur. J. Immunol. 27 (1997), 1303-1307).

T lymphocytes comprise a central part of the immune system in vertebrates. T cell receptors that are clonally distributed antigen receptors are specialized to interact with antigens exclusively on cellular surfaces in association with class I (CD8⁺ cytotoxic T cells) or class II (CD4⁺ helper cells) gene products of the major histocompatibility gene complex (MHC). This so-called MHC-restricted antigen recognition results from stringent selection processes during T cell maturation in the thymus. It is this complex MHC + peptide expressed on the cell surface of specialized cells which contacts the TCR and eventually "activates" the specific T cell population.

In addition, it has long been known that T cells may also react to a multitude of chemical reagents, metal salts and drugs, so-called haptens, in an antigen-specific and MHC-restricted way (Pohlit et al., Eur. J. Immunol. 9 (1979), 681-690). Typical examples of protein-modifying haptens are chemicals such as trinitrochlorobenzene or trinitrobenzene sulfonic acid (TNBS) (Shearer, G.M., Eur. J. Immunol. 4 (1974), 527-533), metal ions or natural compounds but also drugs like penicillins (Koponen et al, J. Allergy Clin. Immunol. 78 (1986), 645-652). Such reagents require binding to carrier proteins to become antigenic.

It was therefore an object of the present invention to provide molecules that allow to study the interactions of certain drugs and the components of the immune system and especially with TCRs as kind of model systems.

It was a further object of the present invention to provide possibilities to make use of the effects of such molecules for patients to benefit of their properties. Especially it is desirable to provide diagnostic tools to identify specific T cells that are able to induce adverse type hypersensitivity reactions and to provide possibilities to prevent certain types of allergic reactions.

In one aspect, the invention therefore provides hapten-modified peptides for use as pharmaceutical compounds and/or diagnostic tools, wherein the peptide contains a backbone of amino acids wherein on at least one of these amino acids a penicillin antibiotic is bound. In a preferred embodiment of the invention the penicillin antibiotic is Penicillin G (Pen G), Penicillin V (Pen V) or Ampicillin (Amp).

Upon initial exposure to a foreign antigen, T cells can differentiate into type I or type II phenotype with different functional capacities. Type 1 cells secrete mainly IFNγ and are involved in cell-mediated adverse reactions. Type 2 cells produce IL4 and drive immediate type of hypersensitivity reactions (O'Garra, A., Immunity 8 (1998), 275). In cases where polarized responses are dominant, the antigen dose appears as one of the critical parameters in determining naive T cell differentiation (Constant et al, J. Exp. Med. 182 (1995), 1591). For peptidic antigens, it has been shown that high antigen doses drive IFNγ production and type 1 differentiation of naive T cells, while low doses of the same antigen lead to IL4 secretion and type 2 differentiation.

Using as module the β-lactam antibiotic Pen G, the inventors could in fact demonstrate that penicillin-specific CD4⁺ T cells react to synthetic MHC class II binding peptides that have appropriate HLA-DR anchor residues and penicilloylated lysin in selected positions along the polyalanine backbone. The modulation of T cell phenotype induced by hapten-specific stimulation was examined using as model β-lactam antibiotics such as Pen G, Pen V and Amp.

It was discovered that the hapten-modified peptides according to the invention are especially suitable when their amino acid backbone consists of 8 to 20 amino acids, preferably 20 to 18 amino acids. In a further preferred embodiment the amino acid backbone contains at least one lysin onto which the antibiotic is bound. In still a further preferred embodiment the hapten-modified peptide contains a tyrosine on the amino acid backbone. The tyrosine is preferably present, since it serves as anchor to the MHC molecules. Once the hapten-modified peptide is anchored to the MHC molecule, it has the right conformation to contact and eventually bind specific TCR.

In the further preferred embodiment the lysin onto which the antibiotic is bound is located in position 3, 5 or 8 calculated from the tyrosine anchor on the amino acid backbone.

The hapten-modified peptides according to the invention are useful as pharmaceutical compounds and/or diagnostic tools. One diagnostic application for the hapten modified peptides is the screening of patients for a predisposition for hypersensitivity reactions against penicillins in general. In a preferred embodiment of the invention PBMCs (peripheral blood mononuclear cells) are isolated from patients with suspected predisposition of allergic reactions. PBMCs are easily purified from the blood of patients. They are stimulated in vitro by addition of the hapten-modified peptides according to the invention and the proliferation of antigen responsive T cells is measured.

In view of the fact that such responsive T cells bind via their TCR to the hapten-modified peptides, such T cells can be detected by an immunoassay using the hapten-modified peptides as capture reagent. Immunoassay protocolls for conducting such assays are known in the art and can easily be adjusted to the present object.

The invention, however, is not to be restricted to such immunoassays but every other possibility to measure proliferation of antigen responsive T cells is also encompassed by the present invention.

Since such proliferating T cells produce IFNγ and IL4 a further preferred possibility to measure proliferation lies in the monitoring of production of these substances.

Since the hapten-modified peptides according to the invention very potently stimulate T cell proliferation a further use of these substances lies in their application as pharmaceuticals for the desensitization of patient suffering from hypersensitivity reactions against penicillins or parts or derivatives thereof. This use for desensitization is a further object of the invention.

It is easily feasible to combine the peptides according to the invention with penicillin haptens, against which the allergic reaction is directed. Therefore, for patients that show hypersensitivity against for example Pen G, Pen G is used as hapten.

The hapten-modified peptides according to the invention can easily be produced in pure form. This enables a safe desensitization without the risk of further adverse reactions to the antibiotic molecule which, once covalently bound to the peptide, looses its chemical reactivity. The production of the hapten-modified peptides also is not expensive and the overall desensitization might become much cheaper as it is at present.

It is also preferred to use parts or derivatives of the penicillins for the desensitization treatment. Such parts of the molecule might be useful for the hyposensitization, but might not be as immunogenic as the complete drug molecule itself. Only parts of the penicillin molecules can therefore provide a possibility of safer and less radical treatments.

Still a further object of the invention is the use of a penicillins according to the invention as pharmaceuticals for the treatment of autoimmune diseases or other pathological situations where IFNγ-mediated effects are involved in the progression of the disease.

As already mentioned above, the specific immune response to penicillins is dose dependent. The inventors could show that penicillins can be used to downmodulate IFNγ expression. Preferably Pen V is used as pharmaceutical blocking the IFNγ production in vivo. This use of Pen V, therefore, is another object of the present invention.

The finding that Pen V can be used to downregulate IFNγ production provides a new and promising possibility for therapy of autoimmune diseases or other pathologies where where IFNγ mediated effects are involved in the progression of the disease or at least improvement of the situation of the patients.

The following examples together with the figures are intended to further illustrate the invention.

### Example

### 1. Antigens

Pen G, Pen V and Amp were obtained from Sigma, St. Louis, MO. PHA was from Murex Diagnostics Ltd., Dartford, UK. The tetanous toxoid peptide 616-630 (VRDIIDDFTNESSQK) was synthesized by continuous-flow solid-phase peptide synthesis. For all antigens, a 10-fold concentrated stock solution was freshly prepared in PBS and added to the wells at the time of experiment.

### 2. Culture media

The complete culture medium used in this study was RPMI 1640 supplemented with 2 mM L-glutamine, 5 x 10⁻⁵ M 2-mercaptoethanol, 1 mM sodium pyruvate and 1 x mixture of non-essential amino acids (Gibco BRL, Paisley, Scotland). The medium was prepared without penicillins or any other antibiotics. For growing T cell clones the complete RPMI medium was used with addition of 5% pooled human AB serum (HS; Swiss Red Cross, Bern Switzerland) and 250 U/ml of recombinant human IL2 (Proleukin, EuroCetus, Ratingen, Germany). EBV-transformed B-lymphoblastoid cell lines were grown in complete RPMI supplemented with 10% heat-inactivated fetal calf serum.

### 3. Primary culture

Donors BAM, CAS and ES had a positive history of penicillin allergy and their clinical data are summarized in Table I. Primary cultures were prepared from freshly isolated PBMC in two parallel 96 well plates. Cells at a concentration of 3 x 10⁵/w were stimulated with 3-fold dilutions of Pen G from 3 mg/ml to 0.1 µg/ml. After 5 days incubation at 37°C in 5% CO₂, the proliferative response was determined, in one of the plates, as the ³H-thymidine incorporation after 16 h incubation with 1 mCi = 37 kBq ³H-thymidine (Dupont, Boston, MA). Cells were harvested on GF/A-filters (Dunn Labortechnik GmbH, Asbach, Germany) and the incorporation was measured in an automatic β-counter (Inotech, Asbach, Germany). The second plate was further expanded in IL2-containing medium for 4 days. Cells were then pooled for any given Pen G concentration, washed and rested in the absence of IL2. After another 4 days, cells were harvested and analysed for TCR expression by cell surface staining and IL4/IFNγ production by intracellular staining.

### 4. T cell clones

T cell clones were generated from Pen G specific T cell lines. Freshly isolated PBMC were stimulated with 1, 0.3, 0.1 mg/ml of Pen G at a density of 2 x 10⁵ cells/well in a final volume of 200 µl of complete RPMI containing 5 % HS in 96-well flat bottom culture plates. After 5 days at 37°C and 5 % CO₂, 25 U/ml of IL2 was added to the wells. After another 4 days the cultures were pooled, rested for 3 days in the absence of IL2 and subjected to a second round of restimulation using irradiated autologous PBMC as APC and the same dose of Pen G given during the first restimulation. After 3 days the different cultures were again expanded in IL2 containing medium and then cloned by limiting dilution. T cell blasts were seeded at 0.3 cells/well in Terasaki plates (Nunc, Roskilde, Denmark) in the presence of 1 µg/ml PHA and 10⁴ irradiated allogeneic PBMC in complete RPMI medium containing 5 % HS and 250 U/ml IL-2. The T cell clones obtained were maintained in culture by periodic stimulation in the presence of irradiated allogeneic PBMC, PHA and IL2.

### 5. EBV-transformed B cells

Autologous EBV-transformed B-lymphoblastoid cell lines (EBV-B cells) were prepared by culturing fresh isolated PBMC in complete RPMI containing 10 % FCS, 30 % supernatant of the EBV-producing marmoset cell line B95-8 (American Type Culture Collection, ATCC, Rockville, MD) and 600 ng/ml cyclosporin A (Sandoz, Basel, Switzerland). After overnight incubation, cells were washed and cultured in complete culture medium.

### 6. Flow cytometric analysis of intracellular IFNγ and IL4 production

Intracellular staining for cytokines was performed following the protocol described by Murphy et al. (12), with few modifications. Briefly, cells were resuspended at 10⁶/ml in 96 well plates in a final volume of 200 µl/w of culture medium and stimulated with PMA at 50 ng/ml plus ionomycin at 500 ng/ml for 5 h at 37°C. Brefeldin A at 10 µg/ml was then added to the wells and incubation was prolonged for another 4 h. Cells were then washed and resuspended in 100 µl/w of PBS before adding an equal volume of 4 % formaldehyde fixative. After fixing for 20 min at room temperature, cells were either stored at 4°C over night or stained immediately for intracellular cytokines. For intracellular staining, all reagents and washes contained 1 % BSA and 0.5 % saponin, and all incubations were at room temperature. Cells were first washed and permeabilized for 30 min. After washing, blocking mouse IgG (Jackson Immunoresearch Laboratories, Inc., West Gove, PA) at a concentration of 300 µg/ml were given to the well for 10 min. Then, PE-conjugated anti human IL4 antibody (Clone 8D4-8, Pharmingen, San Diego, CA) plus FITC-conjugated anti human IFNγ antibody (Clone 4S.B3, Pharmingen) or isotype matched controls (PE- and FITC-conjugated mouse IgG1, Pharmingen) at a final concentration of 5 mg/ml, were directly added to the wells. After 20 min, cells were washed twice in PBS/BSA/saponin and then with PBS/BSA to allow membrane closure. Samples were resuspended in PBS/BSA and analyzed on a FACScan flow cytometer (Becton Dickinson & Co.). Thresholds were set on control stainings, included for every sample; 50,000 cells/sample were acquired. Results were analyzed using Cellquest software. T cells were detected by standard surface staining using the PE-conjugated anti human TCRαβ antibody (Clone BMA03, Immunotech, Marseille, France).

### 7. Cytokine measurement

For cytokine measurement, 10⁵ T cells and 5 x 10⁴ autologous, irradiated EBV-B cells were mixed in 200 µl of complete RPMI-FCS medium containing appropriate Ag concentration. After overnight incubation, supernatants were collected and tested for the concentration of IL2, IFNγ and IL4. IL2 was quantified on a IL2-dependent CTLL line. IFNγ was measured by a sandwich ELISA as described (12). IL-4 was measured using the Duo-Set ELISA kit provided by Genzyme Diagnostics, Cambridge, MA. Eventually, cells were kept in culture for another 24 h and cellular proliferation was measured by thymidine uptake as described above.

### 8. Preferential expansion of PBMC-derived IL4-producing cells upon stimulation with different doses of Pen G

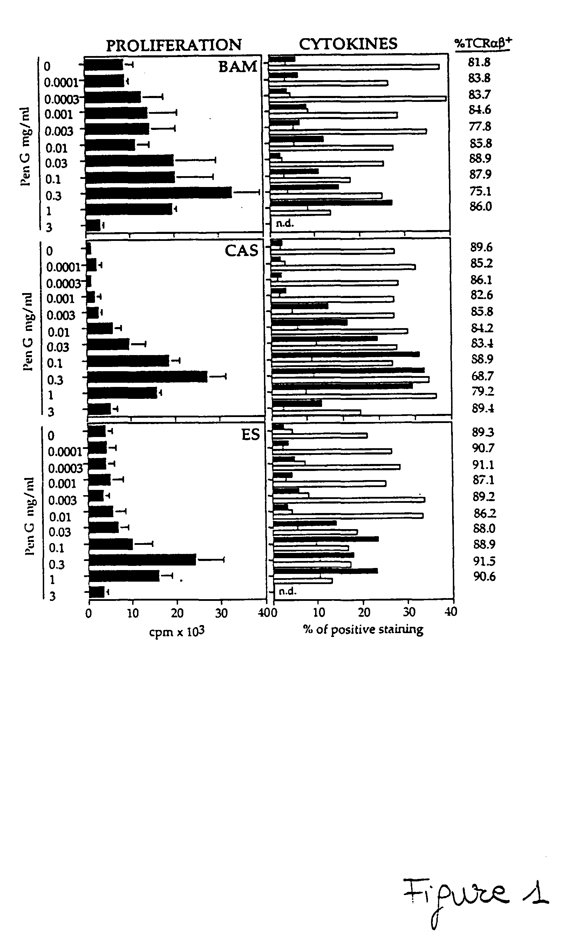

Patients BAM, CAS and ES participating to this study developed allergic reactions after treatment with β-lactam antibiotics and their clinical data are summarized in Table I. At the time of diagnosis they all presented different symptoms after treatment with β-lactams as well as positive LTT in vitro (Table I; ref. 2, 4 and data not shown). The Pen G specificity of their allergic reactions was assessed by RAST and skin tests which gave clear positive results for donors BAM and ES, but were negative for donor CAS. For all the three donors an immediate type of hypersensitivity was diagnosed, associated to delayed type reactions for donor BAM. Primary in vitro cultures were prepared stimulating purified PBMC with different doses of Pen G. The hapten was given in three fold dilutions starting from 3 mg/ml down to 0.1 µg/ml concentration, in two parallel cultures. After 5 days of incubation, the proliferative response was measured in one of the cultures by thymidine incorporation. As shown in Fig. 1, PBMC proliferated in response to Pen G in the range from 1 to 0.01 mg/ml, depending on the donor. The intensity of the response was dose-dependent, with maximal proliferation at 0.3 mg/ml of Pen G.

The second culture was further expanded in IL2-containing medium for 4 days. Cells were then washed and rested in the absence of IL2. After another 4 days, cells were harvested, restimulated with PMA and ionomycin, and analysed for intracellular IFNγ and IL4 expression by flow cytometry. TCRαβ⁺ cells constituted up to 90 % of the cell populations and they were homogeneously represented in the different cell lines (Fig. 1). As shown in the right panels of Fig. 1, upon in vitro stimulation with Pen G, mainly IL4⁺ and IL4⁺IFNγ⁺ cells are expanded, while the growth of IFNγ-producing cells was rather unaffected. Comparing left and right panels of Fig. 1, the expansion of IL4-producing cells directly correlated with the intensity of the proliferative response. This indicating that, in our system, Pen G preferentially induced the recruitment of IL4-producing cells from peripheral blood lymphocytes and the intensity of their expansion depended on the dose of hapten used to induce the primary response in vitro. Interestingly, the preferential expansion of Pen G-specific, IL4-producing cells was associated to immediated type of hypersensitivity reactions in all the patients analyzed (Table I).

### 9. Effect of "hapten doses" on T cell clones

In order to evaluate the role of Pen G doses in inducing functional differentiation of established T lymphocytes, a panel of independent Pen G-specific T cell clones was prepared. PBMC from donors BAM and ES were subjected to two rounds of stimulation, in vitro, with Pen G at concentrations of 1, 0.3 and 0.1 mg/ml in three independent T cell lines. After expansion in IL2-containing medium, each line was independently cloned by limiting dilution. A total of 18 Pen G-specific T cell clones were obtained from donor BAM and 24 from donor ES. The hapten-specific T cell phenotype was induced stimulating each T cell clone with 3-fold dilution of Pen G, from 3 mg/ml to 0.03 mg/ml concentration, in the presence of irradiated autologous EBV-B cells. After 24 h incubation, supernatants were harvested and processed for cytokine measurement, while cells were kept in culture for another 24 h and T cell proliferation was measured by thymidine uptake. We then examined IL4 and IFNγ secretion induced in the presence of the highest and lowest Pen G dose able to trigger cellular proliferation, considering SI values ≥ 2.0 as positive response. Results are reported in Table II.

Clones from donor BAM showed strong proliferation in response to the highest dose of hapten (3 mg/ml), with SI values up to 122.7. For these clones the lowest dose of Pen G capable of triggering cellular proliferation was 0.03 mg/ml and, even at this low concentration, SI values of proliferation wer up to 86.5, indicating the high sensitivity of these reactivities. Phenotypically, BAM clones expressed a strong preference for type 0 or type 1 responses, and half of the clones were able to secrete both IL4 and IFNγ at high and low hapten doses. Interestingly, lower Pen G concentration induced higher level of IFNγ secretion in 14 of the 18 clones analyzed. This dose-dependent cytokine pattern resulted in a hapten-specific modulation of the T cell phenotype, and remarkably, 5 of the clones analyzed were able to shift from type 0 to type 1 responses.

Clones from donor ES resulted less sensitive to the hapten, being unable to proliferate to Pen G at concentrations < 0.3 mg/ml. For these the lowest Pen G doses capable of triggering cellular proliferation were ≥ 0.3 mg/ml with SI never exceeding 8.6. Clones ES mounted type 2 responses but could secrete IL4 only at the highest dose of Pen G tested (3 mg/ml).

Altogether, the hapten-specific T cell phenotypes were independent of the dose of Pen G used during in vitro induction of PBMC but depended on the donor analyzed. Thus, from donor BAM, who suffered of delayed as well as immediate type of hypersensitivity reactions, it was possible to isolate, after two cycles of restimulation with the specific hapten, IFNγ-producing T cell clones. In contrast, from donor ES, characterized by immediated type of hypersensitivity, mainly IL4-producing T cell clones could be isolated. However, the concentration of Pen G was indeed capable of modulating the T cell phenotypes. In fact, very high doses of Pen G were required to induce IL4 production by ES clones, while low doses of hapten induced higher level of IFNγ secretion, resulting in a shift of T cell phenotypes.

### 10. Pen G can modulate the T cell phenotype of fully differentiated hapten-specific T cell clones

To rule out if the antigen-driven phenotype modulation was a peculiar characteristic of the hapten under investigation, we further examined the effect of "antigen doses" on two other CD4⁺ T cell clones.

Clone BAM25 was isolated 2 years ago in our laboratory and has been already described as Pen G-specific (7). clone 1 ES4 derived from stimulation of peripheral blood cells of donor ES with the tetanus toxoid peptide sequence 616-630. The two clones were stimulated in vitro with different concentrations of either the specific Ag or the T cell mitogen PHA and cytokines released in the culture supernatant were measured after 24 h incubation.

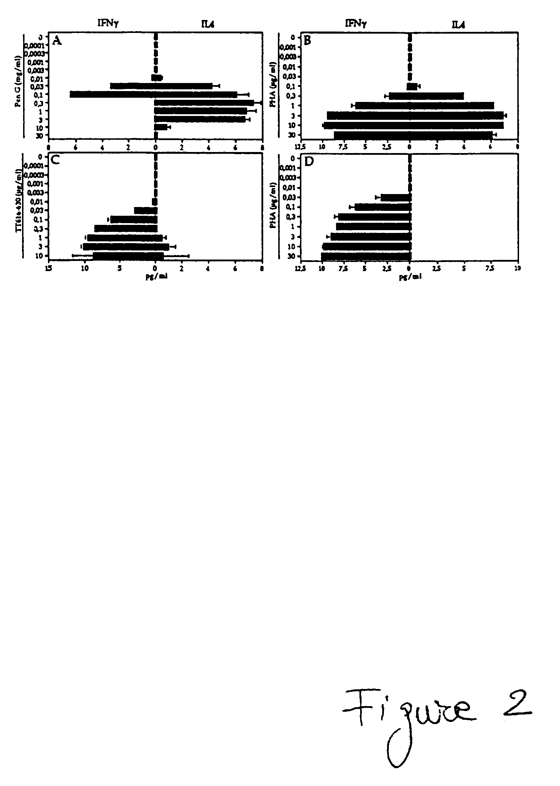

As shown in Fig. 2, the cytokine patterns of clone BAM25 depended on the dose of Pen G used for stimulation. This clone was raised with a dose of Pen G of 1 mg/ml (7) and behaved, in this condition, as a Th2 clone, secreting IL4 but not IFNγ. However, the same clone simultaneously secreted IL4 and IFNγ in the presence of lower doses (< 0.3 mg/ml) of Pen G (Fig. 2A). In contrast, PHA stimulation induced a dose-dependent production of both IL4 and IFNγ (Fig. 2B), indicating the importance of the hapten-specific stimulation in defining Th0 or Th2 profiles. On the other hand, clone 1ES4 secreted IFNγ in response to both the specific antigen as well as the mitogen in a dose-dependent way (Fig. 2C-D).

Thus, the modulation of the T cell phenotype observed seems to be a peculiar characteristic of our hapten system. It appears feasible to drive the cytokine profile of long-term established Pen G-reactive T cell clones, altering the dose of hapten used for in vitro stimulation.

### 11. TCR-independent IFNγ downmodulation by Pen V

The possibility to modulate the phenotype even of fully differentiated T cell clones could certainly introduce new strategies to manipulate immune responses. However, the data presented above apply exclusively to Pen G specificities. In order to find a system of more general application, we also investigated the possibility to use other penicillin derivatives to modulate different antigen-specific immune reactions.

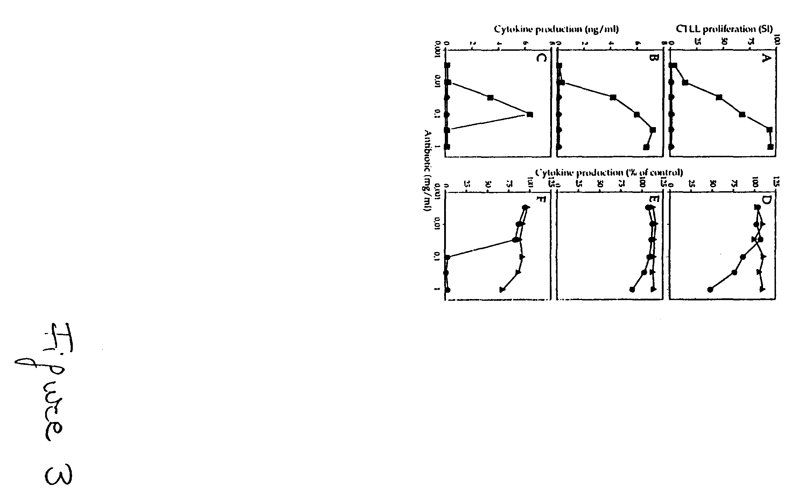

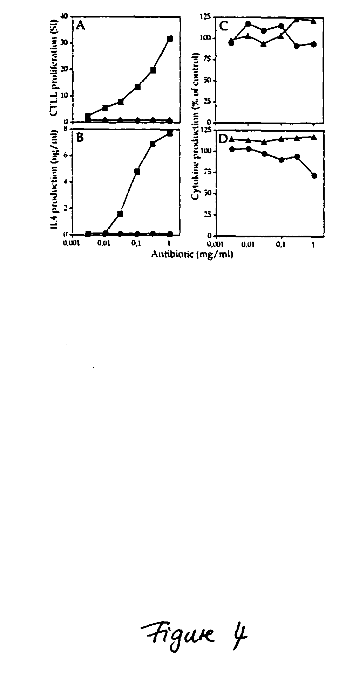

In a first set of experiments we studied the effect of the Pen V and Amp derivatives on the T cell phenotype induced by Pen G on two hapten-specific CD4⁺ cell clones. Upon stimulation with 0.25 mg/ml of Pen G, clone BAM25 secreted IL2, IL4 and IFNγ (Fig. 3A-C), while clone ES5.13 produced IL2 and IL4 (Fig. 4A-B). None of them responded to neither Pen V nor Amp (Fig. 3A-C and Fig. 4A-B, respectively). In the assay, graded concentrations of Pen V and Amp were used together with 0.25 mg/ml of Pen G for stimulating T cell clones and the cytokines secreted in the culture supernatants were measured after 24 h incubation.

Results obtained from clone BAM25 are shown in Fig. 4D-F. Addition of Amp did not affect the Pen G-induced cytokine pattern, except for a slight reduction of IFNγ secretion at high concentration (Fig. 3D-F). In contrast, Pen V reduced the amount of IL2 of about 50 % (Fig. 3D) and completely abrogated IFNγ production at concentrations ≥ 0.1 mg/ml, without affecting IL4 secretion (Fig. 3E-F). The same type of assay, performed on clone ES5. 13, revealed no interference of either Pen V or Amp on Pen G-induced IL2 secretion (Fig. 4C) and only a marginal reduction of IL4 production with high Pen V concentration (Fig. 4D).

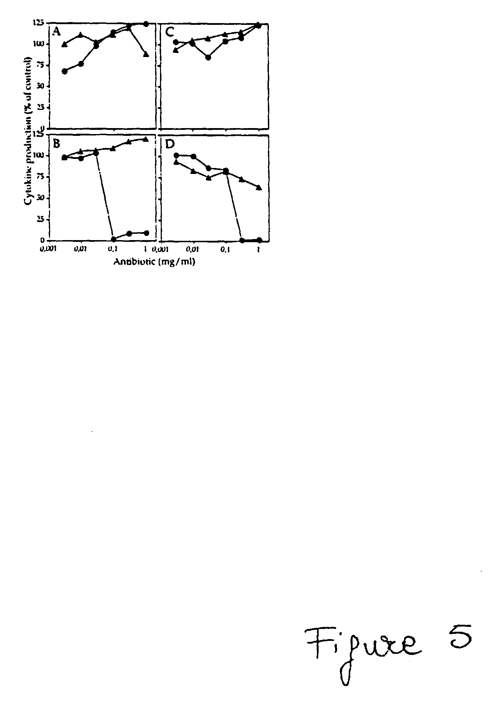

Considering the structural similarities of the different penicillin molecules one might expect that the strong effect on IFNγ downregulation observed with Pen V could be due to altered interactions with the antigen-binding sites of penicillin-specific TCR. In order to determine if this was the case, we performed the same assay described above, with the tetanus toxoid-specific T cell clone 1ES4. Upon stimulation with either the peptide sequence TT 616-630 at 1 µg/ml and PHA at 0.3 µg/ml, this clone has a classical Th1 phenotype (Fig. 2). Surprisingly, even for these reactivities, the addition of Pen V at ≥ 0.1 mg/ml completely abrogated IFNγ secretion in response to either the specific antigen (TT peptide 616-630, Fig. 5B) and the mitogen (PHA, Fig. 5D), while IL2 production was unaffected (Fig. 5A,C). Again, addition of Amp had no effect (Fig. 5).

Thus, a complete inhibition of IFNγ secretion could be induced by addition of Pen V on two T cell clones of totally different specificities, i.e. Pen G (clone BAM25) or tetanus toxoid (clone 1ES4). Therefore, the effect observed is more likely independent of interaction with the TCR, but rather due to a direct interference of Pen V with IFNγ secretion pathways.

### 12. Implications

It has long been known that antigen dose can influence whether cell mediated or humoral responses are elicited and this largely depends on the development of CD4⁺ T lymphocytes producing distinct sets of cytokines (8). In the mouse system, very high doses of antigenic peptides direct the differentiation of naive T cells into IL4-producing cells, while low levels of antigen promote the expansion of IFNγ-producing cells (9, 10). These findings suggested that antigen dose can alter the cytokine synthesis in naive CD4⁺ T cells and even the induction and progression of drug-specific adverse immune responses could be determined at the primary encounter with the hapten.

In the present examples the inventors focussed on the human allergic response to β-lactam antibiotics investigating the influence of hapten doses first, on the recruitment of drug-specific cells from PBMC of allergic donors, secondly, on the induction of T cell phenotype of established T cell clones.

All the donors participating in this study suffered from immediated type of hypersensitivity to β-lactams, and in particular to Pen G. Upon in vitro stimulation with different doses of Pen G we found a preferential recruitment of IL4-producing peripheral blood-derived T cells. This expansion was maximal for high-middle doses of hapten (around 1-0.1 mg/ml concentration) and directly correlated with the proliferative response of the cultures. This suggests that the drug-specific cells contributing to the adverse reactions are mainly of type 2 phenotype, as expected from the clinical characteristics of all the donors. Remarkably, this effect was particularly evident for donor CAS who developed an anaphylactic shock after treatment with β-lactams.

In those experiments the inventors were able to analyze only the contribution of memory T cells to penicillin-specific allergic responses. To rule out how these drug-specific reactions are induced at the very early encounter with the hapten and how hapten concentrations might influence T cell differentiation, it could be certainly interesting to investigate the effect of Pen G on human cord blood-derived naive T cells.

The role of Pen G doses in inducing functional differentiation of established T lymphocytes was analyzed on a panel of 42 independent T cell clones isolated from Pen G-specific after two cycles of restimulation with the hapten. It was found that the concentration of Pen G during in vitro induction does not affect the type of clones isolated, this depending only on the donor studies. However, once T cell clones have been isolated and expanded, the dose of Pen G used for stimulation was, indeed, a critical factor determining their functional, hapten-specific phenotype. In fact, only high doses of Pen G (3 mg/ml) induced IL4 secretion and the type 2 clones isolated behaved as "inert" cells at low hapten concentration. On the other hand, low Pen G concentrations induced higher level of IFNγ secretion; as a consequence, 35% of clones isolated from donor BAM were able to shift their phenotype from type 0 to type 1 (Table II). Remarkably, the phenotype shift was very peculiar for penicillin-specific responses, since it was not observed with a tetanus toxoid-specific CD4⁺ clone (i.e. clone 1ES4) and even not by PHA stimulation (Fig. 2). Moreover, the phenomena observed depended mainly on the modulation of IFNγ secretion as shown in detail for clone BAM25 which could secrete IFNγ only at Pen G concentration ≤ 0.3 mg/ml (Fig. 2).

For a correct interpretation of these data, one should consider how hapten-derived epitopes are formed and how the strength of their interaction with specific TCR is determined, in comparison to nominal peptidic Ag (13, 14). On a given APC, the density of epitopes formed by hapten-modification will depend, at least, on two factors: the number of modifiable sites and the concentration of reactive hapten molecules. At saturation, the density of epitopes will not increase with the concentration of the hapten. On the other hand, multiple modification might interfere with the strength of TCR ligation. We already found that a peptide sequence carrying multiple penicilloyl groups was a poor stimulator compared to a designer penicilloylpeptide carrying only the preferential modified lysin residue, for the same clone (15). Taken together, these considerations suggest that for drug-specific immune responses there is no direct correlation between concentration of hapten molecules, epitope density and strength of TCR engagement.

Several recent studies focus on the identification of factors and signals capable of reversing Th responses (16, 17). In the inventor's system, the phenotype-modulation observed for Pen G-specific immune responses is, most probably, TCR-mediated.

In the last set of experiments presented, it was possible also to identify at least one penicillin derivative capable of inducing a modulation of the T cell phenotype, with a TCR-independent mechanism. In fact, a dose-dependent inhibition of IFNγ production was observed when Pen V was added to manipulate either a Pen G-specific immune response (clone BAM25 in Fig. 3) as well as a tetanus toxoid-specific response or even a mytogen-induced phenotype (clone 1ES4 in Fig. 5). In all these systems a downregulation of IFNγ secretion was observed, with no effect on IL4 production. The broad range of specificities which could be manipulated speaks in favour of TCR-independent mechanisms for Pen V-mediated IFNγ downmodulation. A similar effect has been very recently described for other compounds, such as pirydinil imidazole drugs. One of these compounds, SB203580, selectively induces IFNγ downmodulation in mouse Th1 cells through specific inhibition of p38 MAP kinases (18). It is tempting to speculate that similar targets could be modified also by Pen V.

Taken together, the above data indicate that penicillins can modulate the T cell phenotype of fully differentiated T cell clones in vitro, with TCR-dependent and TCR-independent mechanisms, thus representing potentially new tools for immune intervention. In particular, the possibility to selectively downmodulate IFNγ production via TCR-independent pathways offers a broad application for therapeutic intervention in autoimmune diseases and other pathological situations where a predominant Th1 profile is found.

### Figure 1

Pen G-specific response of PBMC from donors allergic to β-lactams: effect of hapten doses on the recruitment of IL4-producing cells. Purified PBMC from donors BAM, CAS and ES were cultured, in vitro, with different doses of Pen G in two parallel cultures. Proliferative responses measured on the first culture are shown in the left panels; data are expressed in cpm x 10³ and given as mean of triplicates, SD values are indicated. Cells in the second culture were further expanded, then rested in the absence of IL2 and finally harvested, restimulated with PMA and ionomycin, and analysed for IFNγ and IL4 productions by flow cytometry. Data are shown in the right panels and represented as percentage of IL4 (black bars), IFNγ (white bars) and IL4/IFNγ (grey bars) producing cells. For all the cultures the frequency of TCRαβ⁺ cells, reported on the right, was determined by surface staining.

### Figure 2

Pen G can modulate the phenotype of an established CD4⁺ hapten-specific T cell clone. Clones BAM25 (A,B) and 1ES4 (C,D) were analysed for cytokine secretion in response to different concentrations of the specific antigen, Pen G (A) or TT616-630 peptide (C) respectively, and PHA (B,D). IFNγ and IL4 productions are shown in the left and right panels respectively. Data are expressed as pg/ml of secreted cytokines and given as mean of triplicates, SD values are indicated.

### Figure 3

Effect of Pen V and Amp on the Pen G-specific phenotype of clone BAM25. IL2, IL4 and IFNγ productions in response to different concentration of Pen G (square), Pen V (circle) and Amp (triangle) are represented in A, B and C, respectively. In the experiment shown in the right panels, 10⁵ T cells/w and 5 x 10⁴ EBV-B cells/w were stimulated with 0.25 mg/ml of Pen G plus different amount of Pen V or Amp. IL2, IL4 and IFNγ productions, measured after 24 h incubation, are shown in D, E and F, respectively. Data are given as mean of triplicates and represented as % of cytokines secreted in the presence of Pen G only. 100% values were: IL2 80.5 SI of CTLL cells, 1L4 5788 pg/ml, IFNγ 6637 pg/ml.

### Figure 4

Effect of Pen V and Amp on the Pen G-induced phenotype of clone ES5. 13. IL2 and IL4 productions in response to different concentrations of Pen G (square), Pen V (circle) and Amp (triangle) are shown in A and B, respectively. The assay, shown in the right panels, was performed as described in Fig. 3. IL2 and IL4 productions, shown in C and D respectively are given as mean of triplicates and represented as % of cytokines secreted in the presence of Pen G only. 100% values were: IL2 40.3 SI, IL4 5414 pg/ml.

### Figure 5

TCR-independent IFNγ downmodulation by Pen V. Clone 1ES4 was stimulated with the tetanus toxoid peptide sequence 616-630 at 1 µg/ml (A,B) or PHA at 0.3 µg/ml (C,D) plus different doses of Pen V and Amp as described in Figure 3. IL2 and IL4 secretion after 24 h incubation are represented in A, C and B, D, respectively. 100 % values for TT 616-630 were: IL2 137.4 SI of CTLL cells, IFNγ 7497 pg/ml; for PHA: IL2 116 SI, IFNγ 7865 pg/ml.

**Table I**

| **Characteristics of the allergic individuals studied** | | | | | |
|---|---|---|---|---|---|
| Donor | Symptoms | IgE^{a)} | Skin Tests | | Type of hypersensitivity |
| | | | Immediate^{b)} | Late^{c)} | |
| BAM | Urticaria Exanthema | + | - | + | I^{e)} and D^{f)} |
| CAS | Anaphylaxis | - | - | - | I |
| ES | Eodema | + | + | n.t.^{d)} | I |

| | | | | | |
|---|---|---|---|---|---|
| ^{a)} Specific serum IgE as determined by RAST. | | | | | |
| ^{b)} Prick and i.d. tests for Pen G using either benzylpenicilloyl-polylysin or minor-determinant-mixture (MDM:benzylpenicilloyl/Pen G mixture). | | | | | |
| ^{c)} Patch test read after 48 h and 72 h. | | | | | |
| ^{d)} n.t. = not tested. | | | | | |
| ^{e)} I = immediated type hypersensitivity reaction. | | | | | |
| ^{f)} D = delayed type hypersensitivity reaction. | | | | | |

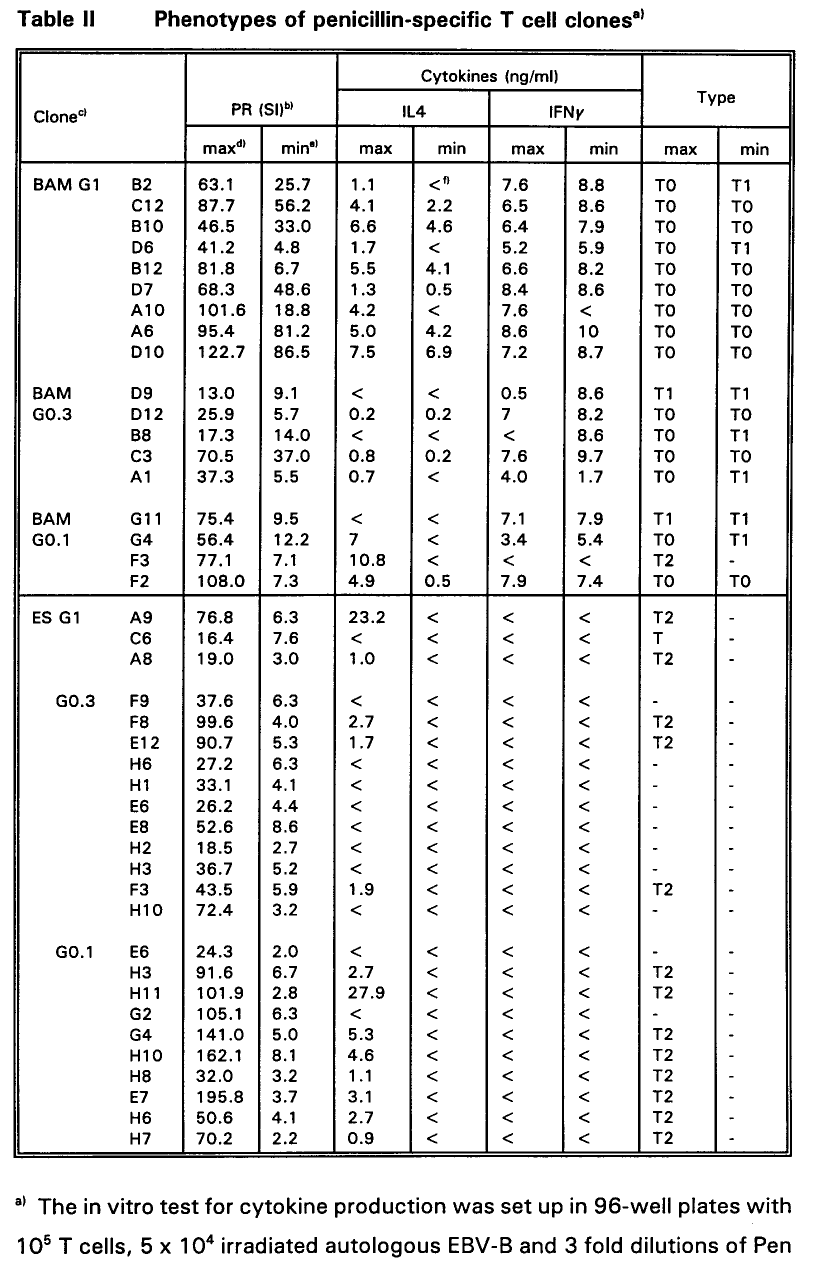

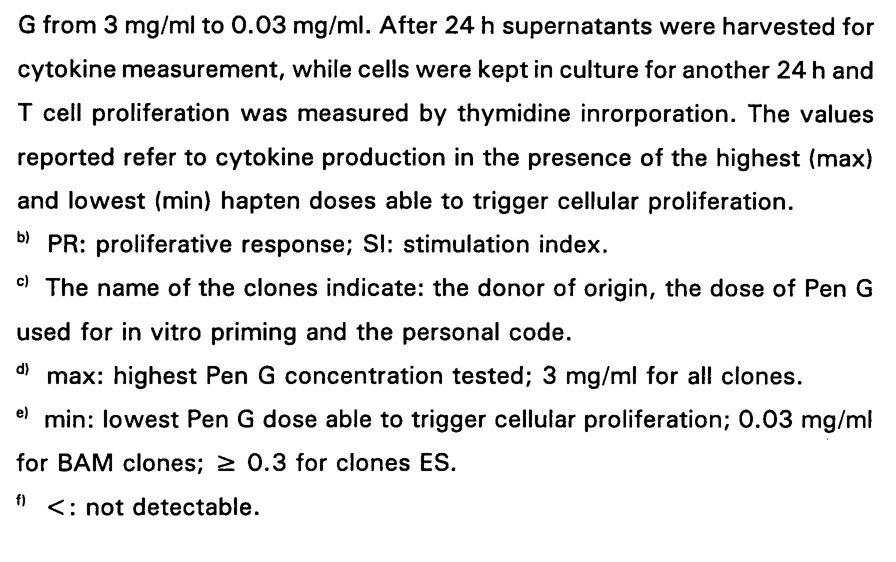

### References

1. DeShazo, R.D. and Kemp, S.F. (1997), Allergic reaction to drugs and biological agents, JAMA 278, 1895.
2. Mauri-Hellweg, D., Bettens, F., Mauri, D., Brander, C., Hunziker, T., Pichler, W.J. (1995), Activation of drug-specific CD4⁺ and CD8⁺ T cells in individuals allergic to sulfonamides, phenytoin and carbamazepine, J. Immunol. 155, 462.
3. Merk, H.F. and Hertl, M. (1996), Immunological mechanisms of cutaneous drug reactions, Seminars in Cutaneous Medicine and Surgery 15, 228.
4. Brander, C., Mauri-Hellweg, D., Bettens, F., Rolli, H., Goldman, M., Pichler, W.J. (1995), Heterogeneous T cell responses to β-lactam-modified self structures are observed in penicillin-allergic individuals, J. Immunol. 150, 2670-2678.
5. Weltzien, H.U., Moulon, C., Martin, S., Padovan, E., Hartmann, U., Kohler, J. (1995), T cell immune responses to haptens. Structural models for allergic and autoimmune reactions, Toxicology 107, 141-151.
6. Padovan, E., Bauer, T., Tongio, M.M., Kalbacher, H., Weltzien, H.U. (1997), Penicilloyl peptides are recognized as T cell antigenic determinants in penicillin allergy, Eur. J. Immunol. 27, 1303-1307.
7. Padovan, E., Mauri-Hellweg, D., Pichler, W.J., Weltzien, H.U. (1996), T cell recognition of penicillin G: structural features determining antigenic specificity, Eur. J. Immunol. 26, 42-48.
8. O'Garra, A. (1998), Cytokines induce the development of functionally heterogeneous T helper cell subsets, Immunity 8, 275.
9. Constant, S., Pfeiffer, C., Woodard, A., Pasqualini, T., Bottomly, K. (1995), Extent of T cell receptor ligation can determine the functional differentiation of naive CD4⁺ T cells, J. Exp. Med. 182, 1591.
10. Hosken, N.A., Shibuya, K., Heath, A.W., Murphy, K.M., O'Garra, A. (1995), The effect of antigen dose on CD4⁺ T helper cell phenotype development in a T cell receptor-αβ-transgenic model, J. Exp. Med. 182, 1579.
11. Murphy, E., Shibuya, K., Hosken, N., Openshaw, P., Maino, V., Davis, K., Murphy, K., O'Garra, A. (1996), Reversibility of T helper 1 and 2 population is lost after long-term stimulation, J. Exp. Med. 183, 901.
12. Elsasser-Beile, U., von Kleist, S., Gallati, H. (1991), Evaluation of a test system for measuring cytokine production in human whole blood cell cultures, J. Immunol. Methods 139, 191-195.
13. Carballido, J.M., Faith, A., Carballido-Perrig, N., Blaser, K. (1997), The intensity of T cell receptor engagement determines the cytokine pattern of human allergen-specific T helper cells, Eur. J. Immunol. 27, 515.
14. Secrist, H., DeKruyff, R.H., Uematsu, D.T. (1995), Interleukin 4 production by CD4⁺ cells from allergic individuals is modulated by allergen concentration and antigen-presenting cell type, J. Exp. Med. 181, 1081.
15. Weltzien, H.U. and Padovan, E. (1998), Molecular features of penicillin allergy, J. Invest. Dermatol. 110, 203-206.
16. Carballido, J.M., Aversa, G., Kaltoft, K., Cocks, B.G., Punnonen, J., Yssel, H., Therstrup-Pedersen, K., de Vries, J.E. (1997), Reversal of human allergic T helper responses by engagement of signalling lymphocytic activation molecule, J. Immunol. 159, 4316.
17. Bellinghhausen, I., Metz, G., Enk, A.H., Christmann, S., Knop, J., Saloga, J. (1997), Insect venom immunotherapy induces interleukin-10 production and a Th2-to-Th1 shift, and changes surface marker expression in venom-allergic subjects, Eur. J. Immunol. 27, 1131.
18. Ricon, M., Enslen, H., Raingeaud, J., Recht, M., Zapton, T., Su, M.S.-S., Penix, L.A., Davis, R.J., Flavell, R.A. (1998), Interferon-γ expression by Th1 effector T cells mediated by the p38 MAP kinase signalling pathway, EMBO J. 17, 2817.

## Claims

1. Hapten-modified peptide for use as pharmaceutical compound and/or diagnostic tool,
**characterized** in that it contains a backbone of amino acids wherein on at least one of these amino acids a penicillin antibiotic is bound.

2. Hapten-modified peptide according to claim 1, wherein the penicillin antibiotic is penicillin G, penicillin V or ampicillin.

3. Hapten-modified peptide according to claims 1 or 2, wherein the amino acid backbone consists of 8 to 20 amino acids, preferably 10 to 18 amino acids.

4. Hapten-modified peptide according to any one of claims 1 to 3, wherein the amino acid backbone contains at least one lysin onto which the antibiotic is bound.

5. Hapten-modified peptide according to claim 4, wherein the antibiotic is bound to amino acids at position 3, 5 or 8 starting from the tyrosine anchor.

6. Use of a hapten-modified peptide according to any one of claims 1 to 5 for the diagnostic determination of a predisposition for hypersensitivity reactions against penicillins or parts or derivatives thereof.

7. Use according to claim 6, wherein PBMC cells are isolated from patients for whom a predisposition for hypersensitivity reactions is to be determined, the cells are stimulated in vitro by addition of the hapten-modified peptide and proliferation of antigen responsive T cells is measured.

8. Use according to claim 7, wherein IFNγ and IL4 expression are measured.

9. Use of a hapten-modified peptide according to any one of claims 1 to 5 for the desensitization of patients suffering from hypersensitivity reactions against penicillins or parts or derivatives thereof.

10. Use of a hapten-modified peptide according to any one of claims 1 to 5 as a pharmaceutical for therapeutic use in autoimmune diseases or other pathological situations where IFNγ-mediated effects are involved in the progression of the disease.

11. Use of a penicillin antibiotic for the preparation of a pharmaceutical for the downregulation of IFNγ production in autoimmune diseases or other pathological situations where IFNγ-mediated effects are involved in the progression of the disease.


SEQUENCE LISTING

<110> Max-Planck-Gesellschaft zur Förderung der Wissensc

<120> Antigenic determinants for drug-specific T lymphocytes
      from drug-allergic patients

<130> 19073PEP Antigenic determinants

<140> EP 98 124 591.3
<141> 1998-12-23

<160> 1

<170> PatentIn Ver. 2.1

<210> 1
<211> 15
<212> PRT
<213> Artificial Sequence

<220>
<223> Description of Artificial Sequence: tetanus toxoid
      peptide 616-630

<400> 1
Val Arg Asp Ile Ile Asp Asp Phe Thr Asn Glu Ser Ser Gln Lys
  1               5                  10                  15